# EUROPEAN PATENT SPECIFICATION

(11) **EP 2 588 063 B1**
(45) Date of publication and mention of the grant of the patent: **27.09.2017**
(21) Application number: 10730011.3
(22) Date of filing: 30.06.2010
(51) Int. Cl.: A61K 8/02, A61K 8/73, A61Q 11/00, A61K 8/81

(54) **FLAVOR RELEASE FROM MULTILAYER FILM DURING BRUSHING**
AROMASTOFFFREISETZUNG AUS EINEM MEHRSCHICHTIGEN FILM WÄHREND DES BÜRSTENS
LIBÉRATION D'ARÔME DEPUIS UN FILM MULTICOUCHE PENDANT LE BROSSAGE

(43) Date of publication of application: 08.05.2013
(73) Proprietor: Colgate-Palmolive Company, New York, NY 10022 (US)
(72) Inventor: BOYD, Thomas, Metuchen New Jersey 08840 (US); GU, Ben, East Brunswick New Jersey 08816 (US); WANG, Wei, East Brunswick New Jersey 08816 (US); LIN, Nora, Basking Ridge New Jersey 07920 (US)
(74) Representative: Wibbelmann, Jobst
(86) International application number: PCT/US2010/040509
(87) International publication number: WO 2012/002946

(56) References cited:
- WO-A1-2005/058265
- WO-A1-2010/114551
- US-A- 3 957 964
- US-A- 5 700 478
- US-A1- 2002 048 553
- US-A1- 2007 148 213
- US-B1- 6 669 929

## Description

### BACKGROUND

This application relates to oral care compositions, and more particularly to compositions comprising a multilayer film entrained in a carrier, in which the multilayer film includes a flavor which can be released during brushing. Such compositions include, for example, dentifrices, mouthwashes and/or oral rinses.

Flavor is an essential component in both dentifrices and oral rinses and is one of the most important features of an oral care product. Flavor also plays a critical role in the success of an oral care product. Good flavor not only enhances product acceptability, but also attracts consumers to buy the product again. Flavor provides not only a sensation of taste on the tongue and of odor in the olfactory center of the nose, but also a key signal which, when perceived by the brain, becomes closely linked to product characteristics and performance to influence the perceptions of the consumer.

Although some currently available products have met with consumer approval, there is still a need in the art for oral care products that can provide enhanced delivery of one or more flavors to the oral cavity of a mammalian subject.

US 6,669,929 describes dentifrice containing functional film flakes.

WO 2005/058265 describes oral and personal care compositions and methods.

US 5,700,478 describes water soluble pressure sensitive muco-adhesive devices for placement in a mucosa-lined body cavity.

US 2007/0148213 describes film-containing compositions.

### SUMMARY

The present invention provides an oral care composition according to claim 1. Preferred features are defined in the dependent claims.

The present invention provides oral care products that employ a multilayer film technology in order to enhance flavor delivery by providing extra flavor and other signals. As a result of using a multilayer film entrained in an orally acceptable carrier, oral care compositions are provided that have enhanced flavor delivery, can deliver different and multiple flavor signals, change the flavor release profile, provide other signals resulting from incorporation of other ingredients and ensure customer compliance with good hygiene practices. Methods of their use also are provided.

In one embodiment, an oral care composition having enhanced flavor release including an orally acceptable carrier containing a first flavor and a multilayer film are provided, the multilayer film including at least a center layer containing a second flavor, the center layer disposed between two outer surface layers, the first and second flavor being the same or different.

In another embodiment, the present invention provides an oral care composition comprising a multilayer film entrained in an orally acceptable vehicle, wherein the multilayer film includes a center layer containing a flavor, the center layer being disposed between two outer surface layers, each outer surface layer including a film forming polymer.

In yet another embodiment, an oral care composition is provided which includes an orally acceptable carrier containing a first flavor and a multilayer film including:
a. a first outer surface layer;
b. at least a first center layer containing a second flavor;
c. a second outer surface layer that can be the same or different from the first outer surface layer, the center layer disposed between the first outer surface layer and the second outer surface layer.

The present invention also provides a method for enhancing flavor release from an oral care composition which includes providing an oral care composition having an orally acceptable carrier containing a first flavor and a multilayer film, the multilayer film including at least a center layer containing a second flavor, the center layer disposed between two outer surface layers, the first and second flavor being the same or different, and applying the oral care composition to an oral cavity of a mammal.

In another embodiment, the invention provides a method of preparing an oral care composition including a multilayer flavored film entrained in a orally acceptable carrier, which comprises:
a. providing an orally acceptable carrier containing a first flavor;
b. adding a multilayer film including at least a center layer containing a second flavor, the center layer disposed between two outer surface layers, the first and second flavor being the same or different; and
c. homogenizing the mixture.

Compositions and methods of this invention afford benefits over compositions and methods known in the art. Such benefits include the ability to provide enhanced flavor delivery systems that provide an intense burst of flavor during the use of the oral care composition, for example during brushing or mouthwash rinsing. The oral care compositions of the present invention also possess enhanced aesthetics and improved stability for delivery of flavor and other sensation signals such as cooling sensation, tingle sensation, sweet and warming signals. Further benefits and embodiments of the present invention are apparent from the detailed description set forth herein.

### BRIEF DESCRIPTION OF THE DRAWINGS

Preferred embodiments of the invention are described in the examples that follow, and illustrated in some of the figures appended hereto, in which:
Figure 1(a) illustrates the flavor release profile of toothpaste A containing multilayer flavored chips.
Figure 1(b) illustrates the flavor release profile of conventional toothpaste B that does not contain any flavored chips.
Figure 2 illustrates a three layer film wherein the center or core layer contains a flavor and the two outer surface layers are without a flavor.

### DETAILED DESCRIPTION

The present invention provides oral compositions and methods, for administration to, or use with a human or other animal subject. Preferably, specific materials and compositions to be used in this invention are, accordingly, pharmaceutically or cosmetically acceptable. As used herein, such a "pharmaceutically acceptable" or "cosmetically acceptable" component is one that is suitable for use with humans and/or animals to provide the desired therapeutic, sensory, decorative, or cosmetic benefit without undue adverse side effects (such as toxicity, astringent taste, irritation, and allergic response) commensurate with a reasonable benefit/risk ratio. The following definitions and non-limiting guidelines must be considered in reading and interpreting the description of this invention set forth herein.

The headings (such as "Background," and "Summary") used herein are intended only for general organization of topics within the disclosure of the invention, and are not intended to limit the disclosure of the invention or any aspect thereof. In particular, subject matter disclosed in the "Background" may include aspects of technology within the scope of the invention, and may not constitute a recitation of prior art. Subject matter disclosed in the "Summary" is not an exhaustive or complete disclosure of the entire scope of the invention or any embodiments thereof.

The citation of references herein does not constitute an admission that those references are prior art or have any relevance to the patentability of the invention disclosed herein.

The description and specific examples, while indicating embodiments of the invention, are intended for purposes of illustration only and are not intended to limit the scope of the invention. Recitation of multiple embodiments having stated features is not intended to exclude other embodiments having additional features, or other embodiments incorporating different combinations of the stated features. Specific examples are provided for illustrative purposes of how to make, use and practice the compositions and methods of this invention and, unless explicitly stated to recite activities that have been done (*i.e*., using the past tense), are not intended to be a representation that given embodiments of this invention have, or have not, been performed.

As used herein, the words "preferred" and "preferably" refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

As used herein, the word "include," and its variants, is intended to be non-limiting, such that recitation of items in a list is not to the exclusion of other like items that may also be useful in the materials, compositions, devices, and methods of this invention. In a similar manner, the description of certain advantages or disadvantages of known materials and methods is not intended to limit the scope of the embodiments to their exclusion. Indeed, certain embodiments may include one or more known materials or methods, without suffering from the disadvantages discussed herein.

As used herein, the term "about" indicates a possible variation of up to 5% in the value.

The oral care compositions of the various embodiments preferably are in the form of a dentifrice. The term "dentifrice" as used throughout this description, denotes a paste, gel, lozenge, gum, or liquid formulation. The dentifrice may be in any desired form, such as deep striped, surface striped, multilayered, having a gel surround the paste, or any combinations thereof. The film contained in the oral care composition may be of any desired shape or structure, including multiple small strips, or one continuous strip.

The expressions "carrier" or "aqueous carrier" as used throughout this description denote any safe and effective materials for use herein. Such materials include, for example, thickening agents, humectants, ionic active ingredients, buffering agents, anticalculus agents, abrasive polishing materials, peroxide sources, alkali metal bicarbonate salts, surfactants, titanium dioxide, coloring agents, flavor systems, sweetening agents, antimicrobial agents, herbal agents, desensitizing agents, stain reducing agents, and mixtures thereof.

The present invention provides oral care compositions comprising a multilayer film entrained in an orally acceptable carrier, wherein the multilayer film comprises at least a flavor containing center layer and at least one outer surface layer containing a film-forming polymer. As used herein, an "oral care composition" refers to a composition for which the intended use can include oral care, oral hygiene, or oral appearance, or for which the intended method of use can comprise administration to the oral cavity. Embodiments of the invention comprise a multilayer film.

As referred to herein, a "film" is a material having a substantially lamellar structure. A "lamellar" structure has, or is capable of having, a size in one or two dimensions (e.g., the x- or y-dimensions) that is substantially greater than the thickness of the structure in a third dimension (e.g., the z-direction). Lamellar structures among those useful herein include those that are substantially planar, layered, or lamelliform. In one embodiment, the lamellar structure is substantially planar, having a size in both the x- and y-dimensions that is substantially greater than the z-direction. In other embodiments, the lamellar structure is non-planar. In one embodiment, a film of this invention comprises a substantially continuous surface that can appear as a substantially flat surface, although in some embodiments the film may be deformed. In such embodiments, the film can have any of a number of shapes, including having a smooth curved surface. The fragments may be of a desired size and may be of regular or irregular perimeter.

In one embodiment of the multilayer film contains at least a center layer containing a flavor and two outer surface layers disposed around the center layer. Each film layer contains at least a film forming polymer, starch film forming agents, a sweetener, a humectant and a surfactant. The center layer further includes a flavor. In another embodiment, the multilayer films of the invention contain a plurality of center layers, each capable of containing a flavor.

The film forming agent used to prepare the multilayer films of the present invention is hydroxypropyl methyl cellulose, methyl cellulose, hydroxypropyl cellulose or mixtures of copolymers thereof. Preferably the cellulose polymer is a low viscosity hydropropylmethyl cellulose polymer (HPMC). When HPMC is used as the film forming agent it is preferred that the HPMC have a viscosity in the range of about 1 to about 40 millipascal seconds (mPa.s) as determined as a 2% by weight aqueous solution of the HPMC at 20° C using a Ubbelohde tube viscometer. Preferably the HPMC has a viscosity of about 3 to about 20 mPa.s at 20° C.

HPMC is available commercially from the Dow Chemical Company under the trade designation Methocel E5 LV. Methocel E5 LV is a USP grade, low viscosity HPMC having 29.1% methoxyl groups and 9% hydroxyproxyl group substitution. It is a white or off-white free-flowing dry powder. As a 2 wt. % solution in water as measured with a Ubbelohde tube viscometer it has a viscosity of 5.1 mPa.s at 20° C.

The hydroxyalkyl methyl cellulose may be incorporated in the layers of the multilayer film in amounts ranging from 25 to 75% by weight and preferably 40 to 55% by weight.

Cold water swellable, physically modified and pregelatenized starches are particularly useful as texture modifier to increase the stiffness of the hydroxyalkyl methyl cellulose multilayer films of the present invention. In the preparation of such starch products, the granular starch is cooked in the presence of water and possibly an organic solvent at a temperature not higher than 10° C higher than the gelatinization temperature. The obtained starch is then dried.

Pregelatinized corn starch is available commercially. A preferred starch is available under the trade designation Cerestar Polar Tex-Instant 12640 from the Cerestar Company. This Cerestar starch is a pregelanterized, stabilized and crosslinked waxy maize starch. It is readily dispersible and swellable in cold water. In its dry form, it is a white free flowing powder with an average flake size no greater than 180 micrometers and 85% of the flakes are smaller than 75 micrometers. It has a bulk density of 0.71g/cm³ (44 lbs/ft.³).

The Cerestar starch has excellent cold storage and freeze-thaw stability. It has a rapid hydration rate and can reach extremely high viscosity without cooking. It has a smooth and creamy texture similar to cook-up starches. It also has excellent paste clarity and a bland flavor.

The pregelatinized starch may be present in the film matrix of the present invention in an amount ranging from about 0 to about 50% by weight and preferably about 10 to about 20% by weight.

Sweeteners also may be incorporated in the layers of the multilayer films of the present invention include both natural and artificial sweeteners. Suitable sweeteners include water soluble sweetening agents such as monosaccharides, disaccharides and plysaccharides such as xylose, ribose, glucose (dextrose), mannose, glatose, fructose (levulose), sucrose (sugar), maltose, water soluble artificial sweeteners such as the soluble saccharin salts, *i.e*., sodium or calcium saccharin salts, cyclamate salts dipeptide based sweeteners, such a L-aspartic acid derived sweeteners, such as L-aspartyl-L-phenylalaine methyl ester (aspartame). D sucralase sweetener is preferred in one aspect of this invention.

In general, an effective amount of sweetener is utilized to provide the level of sweetness desired for a particular film matrix composition, will vary with the sweetener selected. This amount will normally be about 0.01% to about 2% by weight of the composition. However, in some embodiments additional sweetener may be added to the toothpaste directly to ensure the continuous perception of sweetness throughout the use period.

In certain embodiments, it may be desirable to increase the wettability of the multilayer film by adding surfactants. Any orally acceptable surfactant, most of which are anionic, nonionic or amphoteric, can be used. Suitable anionic surfactants include without limitation water-soluble salts of C₈₋₂₀ alkyl sulfates, sulfonated monoglycerides of C₈₋₂₀ fatty acids, sarcosinates, taurates and the like. Illustrative examples of these and other classes include sodium lauryl sulfate, sodium coconut monoglyceride sulfonate, sodium lauryl sarcosinate, sodium lauryl isoethionate, sodium laureth carboxylate and sodium dodecyl benzenesulfonate. Suitable nonionic surfactants include without limitation poloxamers, polyoxyethylene sorbitan esters, fatty alcohol ethoxylates, alkylphenol ethoxylates, tertiary amine oxides, tertiary phosphine oxides, dialkyl sulfoxides and the like. Suitable amphoteric surfactants include without limitation derivatives of C₈₋₂₀ aliphatic secondary and tertiary amines having an anionic group such as carboxylate, sulfate, sulfonate, phosphate or phosphonate. A suitable example is cocoamidopropyl betaine.

Edible non-ionic surfactants are preferred. Examples of edible nonionic surfactants that may be used include polyoxyethylene sorbitan fatty acid esters, polyoxyethylene alkyl ethers, and polyoxyethylene caster oil derivatives. An example of a suitable commercially available non-ionic surfactant that may be used is polysorbate 80, which is a mixture of oleate esters of sorbitol and sorbitol anhydrides, consisting predominantly of the monoester, condensed with approximately 20 moles of ethylene oxide. Polysorbate 80 is sold as Tween^{®} 80 by ICI Surfactants is most preferred for the multilayer films of the present invention. HLB value of the polyoxyethylene sorbitan fatty acid ester should be greater than 10, but should not exceed 20. The inclusion of a surfactant has proven very beneficial in many cases. One or more surfactants are optionally present in a total amount of 0.01% to 10%, for example 0.05% to 5% or 0.1% to 2% by weight of the composition.

The center or core layer of the multilayer films of the present invention includes at least one flavor agent. Flavor agents incorporated in the multilayer films of the present invention are known to the prior art, such as natural and artificial flavors. These flavorings may be chosen from synthetic flavor oils and flavoring aromatics, and/or oils, oleo resins and extracts derived from plants, leaves, flowers, fruits and so forth, and combinations thereof. Representative flavor oils include: spearmint oil, cinnamon oil, peppermint oil, clove oil, bay oil, thyme oil, cedar leaf oil, oil of nutmeg, oil of sage, and oil of bitter almonds. These flavor agents can be used individually or in admixture. Commonly used flavors include mints such as peppermint, artificial vanilla, cinnamon derivatives, and various fruit flavors, whether employed individually or in admixture. Generally, any flavoring or food additive, such as those described in Chemicals Used in Food Processing, publication 1274 by the National Academy of Sciences, pages 63-258, may be used. Generally the flavoring agent is incorporated in the film of the present invention in an amount ranging from 1 to 60% by weight, preferably from 5% to 40%, more preferably 15 to 25% by weight.

The release profile of flavor during brushing or rinsing can be controlled by balancing the thickness and/or composition of the outer surface layers. The thickness of the outer surface layers can vary from 5.1 µm to 101.6 µm (0.2 mils to 4 mils), preferably between 7.62 µm and 19.05 µm (0.3 mils and 0.75 mils).

Without being bound by theory, it is believed that upon contact with saliva or water the multilayer film flavored chips of the present invention begin to slowly disintegrate since each layer and especially the outer surface layers are water erodible. Moreover, when brushed on the teeth, the dentifrice or mouth rinse containing multilayer film flavored chips emit a burst of flavor as the tooth brushing or mouth rinsing also causes the mechanical rupture of the flavored chips with a resulting immediate release of the flavor constituent from the core layer of the multilayer film flavored chips. As a result of incorporating multilayer film flavored chips in a dentifrice or a mouth rinse, the consumer can use oral care compositions that have enhanced flavor delivery. As the flavor of the toothpaste or mouth rinse wanes, the flavor released from the multilayer film flavored chips increases as the flavor from the core layer of the multilayer film chips is released and the consumer experiences an increased flavor sensation long after the flavor from a conventional dentifrice has diminished. Example 1 (b) illustrates this mechanism by indicating a delay period, after which flavor from the multi-layer film releases.

In one embodiment, the flavor of the toothpaste is enhanced by adding to it the multilayer film flavored chips of the present invention, wherein the flavor of the chips is the same as that of the toothpaste.

Extra flavor signals can be delivered by varying the kind of flavor contained in the multilayer films of the present invention. When the flavor of the toothpaste and the one contained in the multilayer film is the same, the intensity of the flavor during brushing increases.

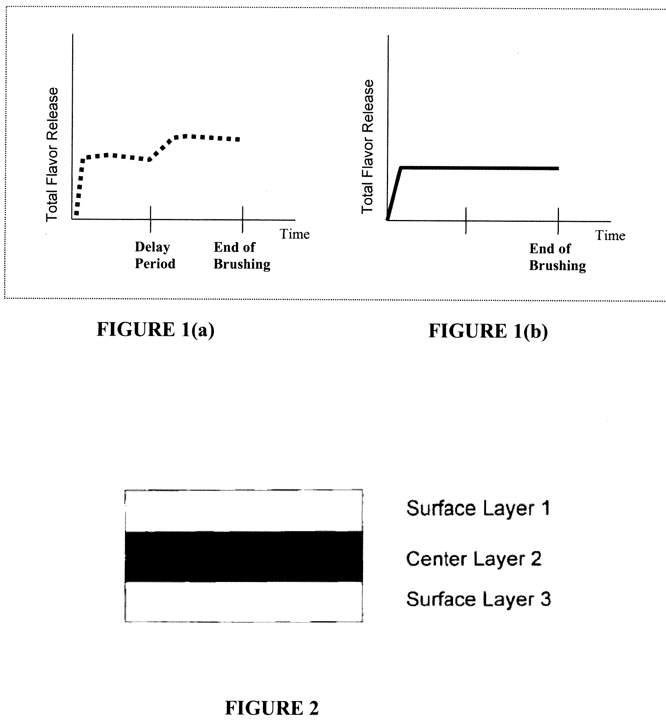

In another embodiment, the perception of flavor can be changed over time by incorporating in the orally acceptable carrier multilayer films having the structures ABA or ABC. Other multilayer film structures are also available such as ABAB or ABCABC. In these film structures, A represents an outer surface layer that includes a film forming polymer selected from the group consisting of hydroxypropylmethyl cellulose, methyl cellulose, hydroxypropyl cellulose and mixtures of copolymers thereof. The outer surface layer A will preferably include corn starch, a sweetener such as D sucralase, a humectant such as propylene glycol and a surfactant such as Tween 80. The surface layer A can include other ingredients that can delay or speed up the flavor release from the center layer. Such ingredients include but are not limited to release modulating polymers such as polyvinyl acetate or hydroxyethyl cellulose. The thickness of the outer surface layers can vary and thus also impact the flavor release from the center layer. When the outer surface layers are thin or about 5.1 µm (0.2 mil) the flavor release from the center layer is accelerated. Conversely when the outer surface layer have increased thickness, for example 101.6 µm (4 mil), the flavor release from the center layer is delayed. Preferably the thickness of the outer surface layers is about 13.97 µm (0.55 mils). The center or core layer B can include the same components as the outer surface layer A, however, layer B also contains a flavor that can be the same or different from the flavor of the orally acceptable carrier. The center layer B can also include other ingredients that can provide additional sensation signals to complement the enhanced flavor signal. When the structure is ABA, the flavor release occurs at different time points following a pattern as discussed below with respect to Figure 1. When the multilayer film structure is ABC, the flavor perception also changes over time. For example, a three-layer ABC multi-layer film structure in a toothpaste can gradually release a citrus flavor from layer C for 30 seconds starting with the beginning of brushing. After the layer C has totally dissolved, then one face of layer B is exposed and can release a second flavor such as high-cooling mint. If layer A has a different rate of dissolving (or is thicker) than layer C, then an increase in release rate of the high-cooling mint in layer B will be noticed after layer A completely dissolves and exposes the second face of layer B.

Varying the composition of the outer surface layers can be used to control the release time of a second flavor signal from the toothpaste or mouth rinse that incorporates the multilayer film flavored chips of the present invention. For example, the flavor release from the center layer can be delayed by adding to the outer surface layers a release modulating polymer such as polyvinyl acetate, hydroxyl ethyl cellulose and the like in an amount effective to delay the release of the flavor from the outer layer for the desired amount of time. For example, by adding from 1 wt% to 5 wt% of polyvinyl acetate, the flavor release may be delayed by 1-5 minutes upon brushing thereby providing a burst of flavor release later in the brushing process. This may be useful to provide a signal to the user that they have brushed sufficiently long.

In another embodiment, varying the thickness of the outer surface layers can be used to control the time of a second flavor signal from the toothpaste that incorporates the multilayer film flavored chips. For example, the thickness of the outer surface layers can vary from 5.1 µm to 101.6 µm (0.2 mil to 4 mil).

In yet another embodiment, the present invention provides a toothpaste that can supply a second flavor signal having an intensity that is different from the intensity of the first flavor signal of the multilayer flavored chips. This can be accomplished by varying the concentration of the flavor in the slurry composition utilized for the center or core film layer of the multilayer flavored chips. For example, the flavor level in the center or core flavor bearing layer can vary from 5% to 40% by weight of the center or core layer composition.

In various embodiments by preparing the multilayer flavored chips with a flavor that is different from that of the toothpaste base, a toothpaste can be obtained which can deliver a second flavor signal that is different from a first signal.

In various other embodiments, multilayer film chips can be prepared to deliver other signals in addition to flavor signals in which the flavor of the core layer is supplemented with one or more agents selected from the groups consisting of a cooling sensation signal, a tingling sensation signal, a sweet sensation signal, a warming signal or a mixture thereof.

Examples of cooling sensation agents that provide a cooling sensation signal include without limitation menthol, WS-3™, WS-5™, WS-23™ and L-menthyl lactate. Examples of tingling sensation agents that provide a tingling sensation signal include without limitation spilanthol and capsiacin. Examples of sweet sensation agents that provide a sweet sensation include without limitation saccharin, sucralose, aspartame, neotame, and acesulfame-K. Examples of warming sensation agents that provide a warming sensation include without limitation cinnamic aldehyde, capsaicin, zeolites, and capsicum oleoresin.

The multilayer film thickness ranges in size from 25.4 µm to 254 µm (1.0 mils to 10 mils) and preferably 38.1 µm to 101.6 µm (1.5 to 4 mils). The dried film of the present invention may then be cut or punched into shaped flakes having a particle size of 0.25mm to 12.7mm (0.01 to 0.50 inches) preferably 2.03mm to 6.35mm (0.08 to 0.25 inches).

When the film is to be used for decorative effect, the film once formed can be punched into various attractive shaped flakes such as hearts, stars, diamonds and circles. The film flakes may be incorporated in the base dentifrice of the present invention at a concentration of about 0.05 to 2.0% by weight and preferably 0.2 to about 0.75% by weight.

The multilayer films of the present invention can be prepared using methods known in the art such as conventional extrusion, aqueous and solvent casting processes. In one embodiment a three layer film is provided. The three layer film can be formed by successively forming the layers A, B, A by casting from a respective slurry and subsequent drying for each layer. For example, the first layer can be cast from a slurry composition including film-forming polymer as a 127 µm (5 mils) layer, and then dried for 15 minutes in an oven at a temperature of 95°C. Then, the second layer can be cast over the second layer from a slurry composition including a film-forming polymer and a flavor as a 381 µm (15 mils) layer, and then dried for 15°C, minutes in the oven at 95°C. Finally, the third layer can be cast over the second layer from a slurry composition including a film-forming polymer as a 127 µm (5 mils) layer, and then dried for 15 minutes in an oven at a temperature of 95°C. Other means to produce 3-layer and multilayer films are well known to the art and include aqueous casting, solvent casting, extrusion, and the like. Films can be made sequentially, such as illustrated above, or formed as a multilayer laminar slurry directly from the component slurries. The laminar slurry would then be dried in a similar manner. Combinations of methods are also possible. For example, extrusion of layers can be followed by solvent casting of different layers. It is not the intent of this description to limit the manner in which the film is crafted. After the multilayer films are prepared, they can be cut into smaller fragments of different shapes or forms, such as for example chips and used for inclusion into a toothpaste or mouthwash base.

In another embodiment, the present invention provides a three-layer film wherein the outer surface layers are water erodible and include a first film forming polymer, while the center layer incorporates a flavor and a second film forming polymer which may be the same or different as the film forming polymer of the center layer.

The overall benefits provided by the multilayer films of the present invention are also illustrated in Figure 1. Figures 1(a) and (b) illustrate typical release profiles of toothpaste A and toothpaste B measured by flavor levels in saliva taken during brushing time. Toothpaste A contains its own flavor but also 3-layer film flavored chips prepared from a 3-layer film having the structure ABA as shown in Figure 2 wherein the outer surface layers A have the same composition and the core layer B is the only layer containing a flavor. Toothpaste B does not contain any flavor chips and has the flavor content of conventional toothpaste. As shown in Figure 1(a), initially the flavor release profile of toothpaste A follows the release profile of a conventional toothpaste B in Figure 1(b). However, after the flavor of the toothpaste base diminishes, as the 3-layer flavored chips start to melt, the flavor from the center layer is released after the Delay Period and the consumer experiences a sudden and intense burst of flavor. As described in more detail below, the intensity of the burst of flavor varies with the amount of flavor in the center layer of the multilayer film chips of the present invention.

Figure 2 illustrates a 3-layer film having the structure ABA wherein the outer surface layers have the same compositions.

In various embodiments, the oral care compositions comprise a plurality of lamellar multilayer film fragments entrained in a carrier. In one embodiment, the composition comprises a multilayer film, wherein the film comprises lamellar fragments of the film material. In one embodiment, the composition comprises a carrier having distributed therein a plurality of lamellar fragments, wherein the fragments comprise a multilayer film wherein the core layer contains a flavor. Such fragments may be of any of a variety of shapes or forms, including semi-solid or solid discrete portions, fragments, particles, flakes, or combinations thereof. In various embodiments, the multilayer film comprises a first plurality of fragments and a second plurality of fragments, wherein the first plurality of fragments differ in composition or appearance from the second plurality of fragments. Such difference in composition or appearance can be in any aspect of the composition of the fragment (*e*.*g*., different film components, different functional material, different formulation colorant), different appearance (*e.g*., shape, color, texture, refractive index, reflective index), or combinations thereof.

In various embodiments, the fragments exhibit perceivable contrast with the carrier. The perceivable contrast can be sensory contrast, such as optical contrast, tactile contrast, taste contrast, or olfactory contrast. In some configurations, optical contrast can be color contrast, or a difference in refractive index or reflective index. In some configurations, color contrast can be imparted by one or more colorants that comprise different components of the composition. In various embodiments, the present invention provides compositions comprising a plurality of film fragments in a carrier, wherein said fragments are visibly discernable. As referred to herein, "visibly discernable" refers to one or more characteristics of a fragment which cause the fragment to have a different physical appearance, preferably to the naked eye, relative to the carrier in which the fragment is entrained. Such characteristics include color, opacity, refractive index, reflective index, size, shape, and combinations thereof.

In various embodiments, the fragments have a non-random shape. In one embodiment, a "non-random" shape is a shape which results from a manufacturing process of shaping, cutting, or other forming process by which a specific shape is imparted to a fragment. In such embodiments, a non-random shape is distinguished from such shapes that result from simple precipitation or grinding of a material. In one embodiment, a "non-random" shape is "repeating," wherein the composition comprises a plurality of fragments have substantially the same shape. Such repeating shape may have any of a variety of forms, and may be selected based on a variety of aesthetic or functional criteria. In certain embodiments, the shape of a film fragment can be a recognizable shape. In certain embodiments, a multilayer film fragment can comprise a nonrandom shape. Such shapes include simple geometric shapes, such as polygons and elliptical shapes, such as triangles, quadrilaterals (such as a square, a rectangle, a rhombus), pentagons, hexagons, oval, and circles. In one embodiment, the repeating shape is a square. Repeating shapes include, in other embodiments, shapes that are representative of figures or animate or inanimate objects, such as stars, hearts, gems, flowers, trees, shamrocks, a letter of an alphabet, numbers, animals, people, and faces. In various embodiments, the composition comprises a single repeating shape. In other embodiments, the composition comprises a plurality of fragments having a plurality of repeating shapes. In one embodiment, the compositions of the present invention comprise a plurality of first multilayer film fragments having a first repeated shape and a plurality of second multilayer film fragments having a second repeated shape, wherein the first repeated shape is different from the second repeated shape.

In various embodiments, the size of the fragments is not critical, and may be determined pursuant to any of a variety of criteria, including manufacturing convenience, affect on visual appearance, surface area, affect on texture in the composition, and combinations thereof. In some embodiments, the multilayer film fragments can be up to about 1 inch (25.4 mm) in length in the longest dimension. As referred to herein, "long dimension" is the dimension of a fragment in length or width (*i.e*., in the x-and y-dimensions, as the fragment is, or is deformed to be, in a planar shape) in a dimension substantially perpendicular to the "thickness" or shortest dimension of the fragment (*i.e*., the z-dimension). It is understood that in various embodiments comprising a plurality of fragments, the fragments may be present in a range of sizes due to a variety of factors, including random variation in size, manufacturing tolerances, and intentional sizing or mixing of the fragments through sieving or similar means. As referred to herein, sizes refer to the average size of fragments in a given plurality of fragments.

In various embodiments, the fragments are from about 0.2 mm to about 15 mm in long dimension. In various embodiments, the long dimension of the fragments is from 0.2 mm to about 10 mm, from about 0.5 mm to about 10 mm, from about 0.8 mm to about 8 mm, from about 0.9 mm to about 5 mm, from about 1.0 mm to about 5 mm, or from about 1.5 mm to about 2.5 mm. In some embodiments, the long dimension of the fragments is at least about 3 mm, and can be from about 6 mm to about 13 mm. In certain embodiments, a plurality of film fragments are greater than about 600 microns in the longest dimension. In certain embodiments, a plurality of film fragments are greater than about 1 millimeter in the longest dimension.

In various embodiments, the fragments of the present invention have a thickness of from about 1 mil (thousandth of an inch, 25.4 microns) to about 3 mils (76.2 microns). In various embodiments, the fragments have a thickness of less than about 4 mils or less than about 100 microns and from about 0.1 mils (2.54 microns) up to about 10 mils (254 microns), from about 0.5 mils (12.7 microns) up to about 5 mils (127 microns), from about 1.4 mils (35.6 microns) to about 2.0 mils (50.8 microns).

In some embodiments, the compositions of the present invention comprise fragments having an aspect ratio of at least about 5:1. As referred to herein, "aspect ratio" of a fragment is the ratio of the diameter of the smallest imaginary sphere that can enclose the object to the diameter of the largest imaginary sphere that can be completely inside the object and tangent to the surfaces of the object. For example, the aspect ratio of a sphere is 1:1; in another example, the aspect ratio of a cylinder that is 2 inches (50.8 mm) long and 1/4 inch (6.35 mm) in diameter is slightly over 8:1; in yet another example, a multilayer film fragment of the present invention that is 1 mil (25.4 microns) in thickness, 1 inch (25.4 mm) in length, and 1 inch (25.4 mm) wide has an aspect ratio of about 1414:1.

In some embodiments, the compositions of the present invention comprise fragments having an aspect ratio of at least about 10:1. In various embodiments, the fragments have an aspect ratio of from 5:1 to 10,000:1, from 5:1 to 500:1, from 10:1 to 1,000:1, from 10:1 to 100:1, from 20:1 to 100:1, or from 25:1 to 35:1.

In various embodiments, the multilayer film comprises a formulation colorant that imparts a color to the multilayer film, the composition, or both. In various embodiments, the film fragments contrast with the carrier, and are white, black, or of any color that is visible against or contrasts with the carrier background. Formulation colorants among those useful herein include non-toxic dyes or pigment, such as, for example, metallic oxide "lakes." In certain embodiments, the colorant is approved for incorporation into a food or drug by a regulatory agency, such as FD&C or D&C pigments and dyes approved by the FDA for use in the United States. Colorants among those useful herein include FD&C Red No. 3 (sodium salt of tetraiodofluorescein), Food Red 17, disodium salt of 6-hydroxy-5-{(2-methoxy-5-methyl-4-sulphophenyl)azo}-2-naphthalenesulfonic acid, Food Yellow 13, sodium salt of a mixture of the mono and disulphonic acids of quinophtalone or 2-(2-quinolyl) indanedione, FD&C Yellow No. 5 (sodium salt of 4-p-sulfophenylazo-1-p-sulfophenyl-5-hydroxypyrazole-3 carboxylic acid), FD&C Yellow No. 6 (sodium salt of p-sulfophenylazo-B-naphtol-6-monosulfonate), FD&C Green No. 3 (disodium salt of 4-{[4-(N-ethyl-p-sulfobenzylamino)-phenyl]-(4-hydroxy-2-sulfoniumphenyl)-methylene}-[1-(N-ethyl-N-p-sulfobenzyl)-Δ-3,5-cyclohexadienimine], FD&C Blue No. 1 (disodium salt of dibenzyldiethyl-diaminotriphenylcarbinol trisulfonic acid anhydrite), FD&C Blue No. 2(sodium salt of disulfonic acid of indigotin), and mixtures thereof in various proportions. In one embodiment, the colorant comprises a water insoluble inorganic pigment, such as titanium dioxide, chromium oxide green, phthalocyanine green, ultramarine blue, ferric oxide, or a water insoluble dye lake. In some embodiments, dye lakes include calcium or aluminum salts of an FD&C dye such as FD&C Green #1 lake, FD&C Blue #2 lake, D&C Red #30 lake or FD&C # Yellow 15 lake. In certain embodiments, a water soluble dye, such as, for example, FD&C Blue #1, is contained within a water-insoluble polymer such as, for example polyethylene such as that found in polyethylene beads (*e.g*., Microblue Spectrabeads, sold by Micropowders, Inc.). In certain embodiments, the multilayer film comprises a dye such as D&C Red #30. In certain embodiments, a white colorant is used, for example titanium dioxide (TiO₂), titanium dioxide coated mica (*e.g*., Timiron), a mineral, or a clay. In certain embodiments, the colorant is a non-bleeding dye. In various embodiments, the multilayer film comprises a colorant at a level of from about from 0.5% to about 20% by weight of the multilayer film, or from about 1% to about 15% by weight of the multilayer film, or from about 3% to about 12% by weight of the film. In one embodiment, the compositions of the present invention comprise a first plurality of multilayer film fragments comprising a first color, and a second plurality of multilayer film fragments comprising a second color. Preferably, the second color is different than the first color.

The multilayer film of the present invention, in various embodiments, disintegrates during use of the composition. In other embodiments, the multilayer film does not disintegrate during use of the composition. In some embodiments, the multilayer film releases a material, such as the flavor, into the carrier. As referred to herein, "disintegrate" refers to physical disruption of the multilayer film or fragment material, so as to produce a film or film fragments of reduced size compared to the original film. Such disruption may be through mechanical, chemical, or physical-chemical means. The disintegration can result, for example, from shearing, grinding, or exposure to elevated temperatures during use. In various dentifrice embodiments of the present invention, such disintegration results from brushing of the composition on the teeth of the subject using the composition. In one embodiment, the film disintegrates so as to release a functional flavor material (as further described herein). In some embodiments, a film fragment can disintegrate into small pieces that are not visually discernable. In some embodiments, the film fragments disintegrate to collectively form a colloid or gel.

In various embodiments, the multilayered films of the present invention may include, in addition to the flavorant and without limitation, other functional actives such as:
A. masking fragrances such as ionones,
B. bacteriostatic or antibacterial agents such as magnolia bark extract, magnolol, honokiol, triclosan, cetyl pyridinium chloride (CPC), chlorhexidine, and the like,
C. metal salts of bismuth, zinc, stannous, copper and the like

In various other embodiments, the multilayer film may comprise, without limitation, and in addition to the flavorant other therapeutic actives. As referred to herein, a therapeutic active is a material that is useful for the prevention or treatment of a physiological disorder or condition. Such disorders or conditions include those of the oral cavity (including the teeth and gingiva), skin, hair, and eyes. The specific therapeutic active is preferably determined according to the desired utility of the composition. In one embodiment, the release of flavor provides a valuable signal to the user that the therapeutic active has enough time to work effectively in the mouth. Such actives include the following:
A. antimicrobial agents, such as triclosan, cetyl pyridium chloride, domiphen bromide, quaternary ammonium salts, sanguinarine, fluorides, alexidine, octonidine, EDTA, essential oils such as thymol, methyl salicylate, eucalyptol and menthol, and the like,
B. non-steroidal anti-inflammatory drugs, such as aspirin, acetaminophen, ibuprofen, ketoprofen, diflunisal, fenoprofen calcium, naproxen, tolmetin sodium, indomethacin, and the like,
C. anti-tussives, such as benzonatate, caramiphen edisylate, menthol, dextromethorphan hydrobromide, chlophedianol hydrochloride, and the like,
D. decongestants, such as pseudoephedrine hydrochloride, phenylepherine, phenylpropanolamine, pseudoephedrine sulfate, and the like,
E. anti-histamines, such as brompheniramine maleate, chlorpheniramine maleate, carbinoxamine maleate, clemastine fumarate, dexchlorpheniramine maleate, diphenhydramine hydrochloride, diphenylpyraline hydrochloride, azatadine meleate, diphenhydramine citrate, doxylamine succinate, promethazine hydrochloride, pyrilamine maleate, tripelennamine citrate, triprolidine hydrochloride, acrivastine, loratadine, brompheniramine, dexbrompheniramine, and the like,
F. expectorants, such as guaifenesin, ipecac, potassium iodide, terpin hydrate, and the like,
G. anti-diarrheals, such a loperamide, and the like,
H. H₂ -antagonists, such as famotidine, ranitidine, and the like; and
I. proton pump inhibitors, such as omeprazole, lansoprazole, and the like,
J. general nonselective CNS depressants, such as aliphatic alcohols, barbiturates and the like,
K. general nonselective CNS stimulants such as caffeine, nicotine, strychnine, picrotoxin, pentylenetetrazol and the like,
L. drugs that selectively modify CNS function such as phenyhydantoin, phenobarbital, primidone, carbamazepine, ethosuximide, methsuximide, phensuximide, trimethadione, diazepam, benzodiazepines, phenacemide, pheneturide, acetazolamide, sulthiame, bromide, and the like,
M. antiparkinsonism drugs such as levodopa, amantadine and the like,
N. narcotic-analgesics such as morphine, heroin, hydromorphone, metopon, oxymorphone, levorphanol, codeine, hydrocodone, xycodone, nalorphine, naloxone, naltrexone and the like,
O. analgesic-antipyretics such as salycilates, phenylbutazone, indomethacin, phenacetin and the like,
P. psychopharmacological drugs such as chlorpromazine, methotrimeprazine, haloperidol, clozapine, reserpine, imipramine, tranylcypromine, phenelzine, lithium and the like.

The amount of medicament that can be used in the films of the present invention can be dependent upon the dose needed to provide an effective amount of the medicament.

In various embodiments, therapeutic agents useful herein include anticaries agents, tartar control agents, antiplaque agents, periodontal actives, breath freshening agents, malodour control agents, whitening agents, antibacterials, steroids, anti-inflammatory agents, vitamins, proteins, conditioning agents, moisturizers, antiperspirant actives, deodorant actives, anesthetics, and mixtures thereof.

In certain oral care embodiments, the multilayer film or the oral care composition may comprise an oral care active, which is useful for the prevention or treatment of an oral care disorder or condition. Oral care actives among those useful herein include abrasives, anticaries agents, tartar control agents, antiplaque agents, periodontal actives, breath freshening agents, malodour control agents, tooth desensitizers, salivary stimulants, whitening agents, and combinations thereof. Active materials among those useful herein are described in U.S. Patent 6,596,298 to Leung et al.

Tartar control agents among those useful herein include dialkali or tetraalkali metal pyrophosphate salts such as Na₄P₂O₇, K₄P₂O₇, Na₂K₂P₂O₇, Na₂H₂P₂O₇ and K₂H₂P₂O₇; long chain polyphosphates such as sodium hexametaphosphate; and cyclic phosphates such as sodium trimetaphosphate. In some configurations, a polyphosphate is a β-phase calcium pyrophosphate, such as disclosed in US Patent 6,241,974 to White, Jr. In some embodiments, the film comprises an anticalculus agent at a level of about 15 to 20% by weight of the film.

Odor reducing agents useful herein include sulfur precipitating agents. Such sulfur-precipitating agents include metal salts, such as a copper salt or a zinc salt. Such salts include copper gluconate, zinc citrate and zinc gluconate. These zinc salts can be used in combination or in addition to the zinc compounds included in the film. In various embodiments, the film comprises sulfur precipitating agents at a level of from about 0.01 to about 30% by weight of film, from about 2% to about 2.5% by weight of film, or about 10% to about 20% by weight of film.

In a certain embodiments, the film and/or oral composition may include a saliva stimulating agent (a "succulent"). Such agents include those disclosed in U.S. Pat. No. 4,820,506 to Kleinberg et al. In some configurations, a saliva stimulating agent can include a food acid such as citric, lactic, malic, succinic, ascorbic, adipic, fumaric and tartaric acids. In various embodiments, the film comprises a saliva stimulating agent at a level of from about 0.01 to about 12 % by weight of the film, from about 1% to about 10% by weight of the film, or from about 2.5% to about 6% by weight of the film. In some embodiments, a saliva stimulating agent can be used in the amelioration of dry mouth.

In certain oral care embodiments, the multilayer film comprises other active materials, such as antibacterial agents such as magnolia extract, triclosan, grapeseed extract, thymol, methyl salicylate, eucalyptol, menthol, hop acids, cetyl pyridinium chloride, (including CPC/Zn and CPC + enzymes) and usnic acid; anti-inflammatory agents such a breath freshening agents (for example zinc gluconate, zinc citrate, zinc chlorite and alpha ionone); tooth desensitizers such as potassium nitrate, desensitizing polymers, and desensitizing minerals; anti-inflammatory agents such as magnolia extract, ursolic acid; aloe, and cranberry extract; vitamins such as pantheon, retinyl palmitate, folic acid, tocopherol acetate and Vitamin A; herbs or herbal extracts such as rosemary, oregano, chamomilla recutita, mentha piperita, salvia officinalis, orcommiphora and myrrha; proteins, such as milk proteins and enzymes such as peroxide-producing enzymes, amylase, plaque-disrupting agents such as papain, glucoamylase, glucose oxidase, and "next generation" enzymes; whitening agents such as hydrogen peroxide, urea peroxide and phosphate salts; medicinals, such as aspirin (acetyl salicylic acid), caffeine, and benzocaine; probiotics; abrasives such as silicas (including high cleaning silica); anti-caries agents such as stannous salts (e.g., stannous fluoride) or amino fluoride; a nitric oxide synthase inhibitor such as guanidinoethyldisulfide; calcium; anti-attachment ingredients, such as polyvinylphosphonic acid; preservatives such as Solbrol^{®} (Bayer Chemicals AG);silicones; chlorophyll compounds, anti-leukoplakia agents such as beta-carotene; anti-oxidants such as Vitamin E; and combinations thereof. In some embodiments, the films comprise such active materials at a concentration of about 0.01 to about 30% by weight of film, from about 2% to about 25% by weight of the film, or from about 10% to about 20% by weight of film.

In certain embodiments, the multilayer film and/or oral care composition includes a preservative. A preservative can be added in amounts from about 0.001 wt % to about 5 wt %, preferably from about 0.01 wt % to about 1 wt % of the multilayer film. Non-limiting examples of preservatives include sodium benzoate and potassium sorbate.

The compositions of the present invention comprise a carrier in which a multilayer film, or fragments, is entrained. As referred to herein, a "carrier" is any material or composition in which a multilayer film can be entrained and is suitable for administration or application to the human or animal subject to whom the composition is administered or applied. As referred to herein, "entrained" refers to the embedding or suspension of a multilayer film in a carrier. In various embodiments comprising a plurality of fragments, such fragments may be entrained by embedding, suspension, dispersion or other distribution of the fragments in the carrier. In various embodiments, the fragments are distributed substantially homogenously throughout the carrier. In other embodiments, the fragments are not distributed homogenously in the carrier. In certain embodiments, the distribution of a plurality of multilayer film fragments is substantially isotropic within the carrier. Dentifrice compositions that include a plurality of film fragments dispersed or suspended in a carrier are commercially available under the tradename Max Fresh® or Max White®, from Colgate-Palmolive Company, New York, N.Y.

The compositions of the embodiments may be described as comprising two phases, wherein one phase comprises a carrier and a second phase comprises the aforementioned film or fragment. The term "phase" as used herein denotes a physical phase as understood in the physical and material sciences, *i.e*., a portion of a material whose properties and composition are uniform. However, a phase as used herein can be discontinuous, *i.e*., a phase can comprise a plurality of separate components. For example, a plurality of polymer film fragments of identical composition is considered to comprise a single phase. In some embodiments, a multilayer film fragment can be entirely embedded within the material comprising the first phase, or totally or partially exposed on the surface of the first phase. For example, if the composition is a dentifrice comprising both a gel and multilayer film fragments, a film fragment can be totally surrounded by the gel, or partially or totally exposed on the surface of the gel. In certain embodiments, compositions comprise more than two phases. Such multi-phase compositions include those having two carriers, each of which contributes a phase to the composition, in addition to film fragments as described herein. Other multi-phase compositions include those having a single carrier and two or more pluralities of fragments, wherein the pluralities of fragments have differing compositions.

In various embodiments, the carrier is a liquid, semi-solid or solid. A "liquid" can be a liquid of low or high viscosity. A liquid can be a liquid such that flow is imperceptible under ambient conditions. For example, a soap, such as an ordinary bar of hand soap, can be considered a liquid herein. A liquid can be a thixotropic liquid. A "semi-solid" as used herein can be a gel, a colloid, or a gum. As used herein, semi-solids and liquids are fluids distinguished on the basis of viscosity: a semi-solid is a high viscosity fluid, while a liquid has lower viscosity. There is no definitive dividing line between these two types of fluids. A semi-solid can, in certain embodiments, have a viscosity as high as thousands of mPa·s. Carriers among those useful herein include liquids, pastes, ointments, and gels, and can be transparent, translucent or opaque.

In certain embodiments, the compositions of the present invention are oral care compositions, suitable for administration to the oral cavity. Such compositions include dentifrices, mouthwashes, mouth rinses, dental gels, lozenges, beads, gums, oral strips, mints, liquid toothpastes, sprays, paint-on gels, lip balms, whitening strips, breath strips, oral chews, and combinations thereof. An oral care composition disclosed herein can be used, for example, for cavity prevention, whitening, plaque prevention or reduction, gingivitis prevention or reduction, tartar control, sensitivity prevention or reduction, or breath malodor prevention or reduction, and stain prevention.

The specific composition of the carrier preferably depends on the intended use of the composition. In various embodiments, the carrier is aqueous, comprising from about 5% to about 95% water or from about 10% to about 70% water. In other embodiments, the carrier is substantially non-aqueous. In a dentifrice carrier, water content can be from about 5% to about 70%, from about 10% to about 50%, or from about 20% to about 40%. When the presence of water will cause the film to disintegrate, it is particularly preferred that the dried film contain no free water, in which the amount of water is substantially 0%, or negligible.

The carrier may comprise any of a variety of materials, including emulsifiers, thickeners, fillers, and preservatives. In some embodiments, the carrier may include a functional or active material, such as those described above. In some embodiments, the carrier comprises the same functional material as the film.

In one embodiment, the carrier is suitable for use as a dentifrice. In some embodiments, the carrier comprises a humectant, such as glycerine, sorbitol or an alkylene glycol such as polyethylene glycol or propylene glycol. In some configurations, the carrier comprises a humectant at a level of from about 10% to about 80% by weight, or about 20% to about 60% by weight of the composition. Carrier compositions among those useful herein are disclosed in U.S. Patents 5,695,746 to Garlick, Jr., et al., and 4,839,157 to Mei-King Ng et al.

In various dentifrice embodiments, the carrier comprises thickeners, gelling agents or combinations thereof. Thickeners or gelling agents useful herein include inorganic, natural or synthetic thickeners or gelling agents. In some configurations, the carrier comprises the thickener and gelling agent at total levels of from about 0.10% to about 15% by weight, or from about 0.4% to about 10% by weight of the composition. Examples of thickeners and gelling agents useful herein include inorganic thickening silicas such as: an amorphous silica, for example Zeodent^{®} 165 (Huber Corporation); Irish moss; iota-carrageenan; gum tragacanth; or polyvinylpyrrolidone. In certain embodiments, the carrier comprises a polishing agent, such as a silica, a calcined alumina, sodium bicarbonate, calcium carbonate, dicalcium phosphate or calcium pyrophosphate. In various embodiments, the carrier can be a visually clear composition.

In various dentifrice embodiments, comprising a visually clear carrier, the composition comprises at least one polishing agent. Polishing agents among those useful herein include collodial silica, such as, for example, Zeodent^{®} 115 (Huber Corporation), and alkali metal aluminosilicate complexes (*i.e*., a silica comprising alumina). In some configurations, a polishing agent can have a refractive index close to that of a gelling agent combined with water and/or humectant. In various embodiments, the carrier comprises the polishing agent at a level of from about 5% to about 70% by weight of the composition.

In certain dentifrices, the carrier comprises a surfactant or mixture of surfactants. Surfactants among those useful herein include water-soluble salts of at least one higher fatty acid monoglyceride monosulfate, such as the sodium salt of the monsulfated monoglyceride of hydrogenated coconut oil fatty acids; cocamidopropyl betaine; a higher alkyl sulfate such as sodium lauryl sulfate; an alkyl aryl sulfonate such as sodium dodecyl benzene sulfonate; a higher alkyl sulfoacetate; sodium lauryl sulfoacetate; a higher fatty acid ester of 1,2-dihydroxy propane sulfonate; and a substantially saturated higher aliphatic acyl amides of a lower aliphatic amino carboxylic acid, such as those having 12 to 16 carbons in the fatty acid, alkyl or acyl radicals; and mixtures thereof. Amides can be, for example, N-lauroyl sarcosine, and the sodium, potassium, and ethanolamine salts of N-lauroyl, N-myristoyl, or N-palmitoyl sarcosine. In various embodiments the carrier comprises the surfactant at a level of from about 0.3% to about 5% by weight of composition, or about 0.5% to about 3% by weight of composition.

The present invention also provides methods for making a dentifrice carrier. In one embodiment, water and at least one humectant are dispersed in a conventional mixer until a first homogeneous gel phase is formed. A polishing agent is added into the first homogeneous gel phase. The first homogeneous gel phase and the polishing agent are mixed until a second homogeneous gel phase is formed. A thickener, flavorant and surfactants are added to the second homogeneous gel phase. These ingredients are mixed at high speed under vacuum of about 20 to 100 mmHg.

The compositions of the present invention are preferably stable under normal conditions of storage. As referred to herein, "stable" refers to the lack of significant adverse effect on one, and preferably all, compositional attributes such as appearance, flavor, rheology, and chemical composition of the composition. Preferably, stability in the present compositions includes the compositional and physical stability of multilayer films (including fragments, if any) in the composition. In various embodiments a composition comprising a multilayer film is stable upon storage at ambient temperature for at least about two years. It is understood, however, that in some embodiments, an otherwise stable multilayer film can disintegrate during use (as discussed above), for example, during toothbrushing using a dentifrice composition.

In certain embodiments, a composition can comprise, in addition to film fragments as described herein, two or more carriers, each of which contributes a phase to the composition. Such a composition can be stable to color bleeding. For example, a composition can include film fragments and a striped dentifrice such as that disclosed in US Patent 6,315,986 to Wong et al. In certain embodiments, the film fragments can be of different color(s) than the stripe(s) for enhanced aesthetic appeal.

The dentifrice composition conventionally includes thickening agents that provide the dentifrice with the required rheological properties, so that the dentifrice can be stored in a dispensing container over a period of time and thereafter reliably dispensed therefrom by the user. The dentifrice preferably should have the correct viscosity not only to be dispensed but also to exhibit an acceptable consistency within the mouth during tooth brushing. Typical thickening agents include modified celluloses, such as carboxymethyl cellulose (CMC), and other polysaccharide or gum components.

The polysaccharide thickening agent may comprise at least one of xanthan gum and hydroxyethyl cellulose. The polysaccharide thickening agent typically consists of at least one of xanthan gum and hydroxyethyl cellulose. Preferably, the polysaccharide thickening agent consists of xanthan gum which is present in an amount of from 0.1 to 1.5 wt % based on the weight of the composition, preferably from 0.5 to 1 wt % of the composition. However, minor amounts of additional thickeners may be present, for example carrageenan, gum tragacanth, starch, polyvinylpyrollidone, hydroxyethypropyl cellulose, hydroxybutyl methyl cellulose, hydroxypropyl methylcellulose, hydroxyethyl cellulose, sodium carboxymethyl cellulose (sodium CMC) and colloidal silica. In one embodiment, the thickener concentration ranges of 0.1 wt. % to 5 wt. % based on the weight of the composition. In another embodiment, the thickener concentration ranges of 0.5 wt. % to 2 wt. % based on the weight of the composition.

The present invention also provides processes for making compositions comprising a multilayer film entrained in a carrier. In various embodiments, a plurality of fragments of the film matrix of the present invention are combined with a carrier. In some configurations, a carrier and a plurality of multilayer film fragments can be mixed. In some configurations, the mixing can comprise slow stirring. In one preferred embodiment, the process for making the composition comprising a carrier having distributed therein a plurality of lamellar fragments of the multilayer films of the present invention includes:
(a) providing the orally acceptable carrier;
(b) adding lamellar fragments of the multilayer flavored film to the orally acceptable carrier to form a mixture; and
(c) homogenizing the mixture.

The term "homogenizing" as used herein refers to the admixture of the fragments and the carrier so as to attain a substantially homogeneous distribution of fragments in the carrier. It should be noted, however, that the resulting composition still retains two-phase composition characteristics. Homogenizing may be accomplished using any of a variety of conventional homegenizers.

In another method, the film is added to a component of the orally acceptable carrier (e.g., to a humectant for a dentifrice). The remainder of the carrier then may be made, and the mixture of film then added to the carrier.

Certain embodiments described herein also provide methods for administering oral compositions including multilayer films for flavor release to a human or animal subject. As referred to herein, "administering" refers to any method by which a composition is applied on or administered to the subject. In various embodiments, the administration is topical, wherein the composition is applied to an external surface of the subject, such as to a surface of the oral cavity (*e.g*., teeth, gingival, and tongue. The specific route and method of administration will depend, of course, on the intended use of the composition.

In various embodiments, the present invention provides methods for administering enhanced flavor containing oral compositions to a human or animal subject in need thereof, comprising topically applying to the subject a composition comprising a multilayer film for enhanced flavor release entrained in a carrier. In one embodiment, the method additionally includes disrupting the multilayer film after topically applying the oral composition including the multilayer film. Such disruption may be accomplished by any of a variety of methods, including chemical and/or mechanical means. Chemical means include degradation of the multilayer film by contact with water or a material present at the site of administration (*e.g*., saliva in an oral care application). Physical means include agitation, grinding, and shear forces produced by application of physical energy to the composition during use (*e.g*., brushing in a dentifrice application).

In various embodiments, the present invention provides methods for the treatment of an oral care condition. As referred to herein, an "oral care condition" is any disorder or condition which can be prevented or treated by administration of a composition to the oral cavity, including disorders or conditions of the teeth, oral mucosa, gingiva and tongue. Such conditions include caries, gingivitis, periodontitis, and cosmetic conditions such as yellowing and malodour.

The embodiments described herein can be further understood by reference to the following non-limiting examples.

The examples and other embodiments described herein are exemplary and not intended to be limiting in describing the full scope of compositions and methods of this invention. Equivalent changes, modifications and variations of specific embodiments, materials, compositions and methods may be made within the scope of the present invention, with substantially similar results.

### EXAMPLES

### Example 1 (outside the scope of the invention)

This example illustrates a three-layer film containing a flavor containing center layer disposed between two outer surface layers. Each film layer contains hydroxyl propyl methyl cellulose ("HPMC") as the film-forming polymer and other excipients also present in the center layer as shown in Table 1 below. The center or core layer also includes a flavor.

**TABLE 1**

| **Ingredient** | **Film Slurry wt.%** |
|---|---|
| **Surface Layer 1** | |
| Water | 83.50 |
| HPMC E5 | 12.30 |
| Corn Starch | 2.20 |
| Dsucralase Sweetner | 1.00 |
| Propylene Glycol | 0.60 |
| Tween 80 | 0.40 |

| **Center Layer 2** | |
|---|---|
| Water | 77.50 |
| HPMC E5 | 12.30 |
| Corn Starch | 2.20 |
| Dsucralase Sweetner | 1.00 |
| Propylene Glycol | 0.60 |
| Flavor | 6.00 |
| Tween 80 | 0.40 |

| **Surface Layer 3** | |
|---|---|
| Water | 83.50 |
| HPMC E5 | 12.30 |
| Corn Starch | 2.20 |
| Dsucralase Sweetner | 1.00 |
| Propylene Glycol | 0.60 |
| Tween 80 | 0.40 |

Layer 1 was cast at 127 µm (5 mils) then dried in a 95°C oven for 15 minutes. Layer 2 was cast over layer 1 at 381 µm (15 mils) then dried in the same manner. Layer 3 was cast at 127 µm (5 mils) and the final composition dried at 95°C for another 15 minutes. The thickness of dried film was about 69.85 µm (2.75 mils) with 41.91 µm (1.65 mils) for the center flavored layer and 13.97 µm (0.55 mils) for two surface layers. The film was smashed to pieces by using IKA-WERKE (MF 10 basic). Chips of size between 12 and 20 meshes were collected for making products, such as, for example, tooth paste and mouthwash.

### Example 2

This example illustrates how flavor release is delayed from the multilayer films of this invention. A multilayer film is prepared as in Example 1 above, except that polyvinyl acetate ("PVA") is added to surface layers 1 and 3 as shown in Table 2 below.

**TABLE 2**

| **Surface Layers 1 and 3** | |
|---|---|
| Ingredient | With PVA |
| Water | 82.17 |
| HPMC E5 | 7.20 |
| HPMC E50 | 1.50 |
| Kollicoat 30D (PVA) | 3.33 |
| Corn Starch | 2.00 |
| Carbopol 971P | 1.20 |
| TiO₂ | 0.70 |
| PG | 1.40 |
| Tween 80 | 0.50 |
| Total | 100.00 |

The amount of PVA shown in Table 2 is sufficient to reduce the swelling speed of the multilayer film of Example 1 and increase the dissolution time from less than 2 minutes to from 3 minutes to 4 minutes.

### Example 3 (outside the scope of the invention)

This example illustrates toothpaste formulations. Toothpaste A contained 3-layer flavored chips prepared as in Example 1. Toothpaste B did not contain any flavored chips as shown in Table 3 below.

**TABLE 3**

| Ingredient Name | Toothpaste A | Toothpaste B |
|---|---|---|
| | | |
| Polyethylene Glycol 600 | 1.00 | 1.00 |
| Sodium CMC - 40PM | 0.50 | 0.50 |
| Sodium Saccharin | 0.35 | 0.35 |
| Sodium Fluoride | 0.32 | 0.32 |
| Sorbitol (70% solution) | 68.00 | 68.00 |
| Purified Water | 9.27 | 10.07 |
| Pigment Blue# 15 | 0.01 | 0.01 |
| Silica Zeodent 114 | 8.00 | 8.00 |
| Silica Zeodent 165 | 8.00 | 8.00 |
| Flavor | 1.00 | 1.00 |
| Cocaamidopropyl Betaine | 1.25 | 1.25 |
| Sodium Lauryl Sulfate | 1.50 | 1.50 |
| 3-Layer Flavored Chip | 0.80 | -- |
| Total | 100.00 | 100.0.0 |

The flavor release profile of toothpaste A which contained 3-layer flavored chips followed the pattern of Figure 1. Figures 1(a) and 1(b) illustrate the difference between toothpaste A which included 3-layer flavored chips and toothpaste B which did not. It is readily seen that toothpaste A had a higher flavor intensity profile than toothpaste B. Moreover, the flavor signal generated by the flavor released from the multilayer chips of toothpaste A provided a second more intense flavor signal that occurred during brushing after the flavor of the toothpaste diminished.

With reference to Example 3, toothpaste A which contained 3-layer film flavored chips followed the flavor release profile of Figure 1(a). As illustrated in Figure 1(a), initially, the flavor release profile of toothpaste A as measured by flavor levels in saliva followed the same release profile as that of conventional toothpaste B, and as seen in Figure 1(b), which contained only 1% by weight flavor. After a period of time, as the flavor from the toothpaste A was consumed, the flavor from the 3-layer film flavored chips was released and the consumer experiences a burst of flavor due to the release of the 6% by weight flavor from the 3-layer film flavored chips which lasted throughout the entire brushing experience. The burst of flavor released from the 3-layer flavored chips contained in toothpaste A continued after the initial flavor release thus enhancing the flavor delivery throughout the entire brushing experience.

### Example 4 (outside the scope of the invention)

This example illustrates mouthwash formulations. Three-layer flavored chips prepared as in Example 1 are added to mouthwash A. Mouthwash B does not contain any chips as shown in Table 4 below.

**TABLE 4**

| Ingredient Name | Mouthwash A | Mouthwash B |
|---|---|---|
| Purified Water | 70.51 | 71.01 |
| Sorbitol (70% solution) | 15.00 | 15.00 |
| Glycerin | 5.00 | 5.00 |
| Kelcogel CG-LA | 0.05 | 0.05 |
| Sodium Fluoride | 0.05 | 0.05 |
| Anhydrous Disodium Phosphate | 0.05 | 0.05 |
| Ethyl Alcohol | 6.00 | 6.00 |
| Gantrez BF 96 Solution | 1.50 | 1.50 |
| Sodium Methyl Cocoyl Taurate | 0.25 | 0.25 |
| Menthol Levo | 0.05 | 0.05 |
| Sodium Saccharin | 0.02 | 0.02 |
| Triclosan | 0.03 | 0.03 |
| 38% Na20 Caustic Soda | 0.10 | 0.10 |
| 3-Layer Film Chip | 0.50 | -- |
| 35% Sodium Lauryl Sulfate | 0.75 | 0.75 |
| Optacool C | 0.03 | 0.03 |
| Flavor | 0.10 | 0.10 |
| FD&C RedNo.40 | 0.01 | 0.01 |
| Total | 100.00 | 100.00 |

The flavor release profile of mouthwash A which contains 3-layer flavored chips follows the pattern of Figure 1(a). Mouthwash A provides the additional flavor signal and/or more intense flavor intensity, while mouthwash B does not.

### Example 5A (outside the scope of the invention)

This example shows how to control the time of a second flavor signal of a toothpaste A. A multilayer film is prepared as in Example 1 above, except that the thickness of the outer surface layers is increased to 101.6 µm (4 mils), an amount effective to control the time of a second signal of toothpaste A to the desired time of 3-4 minutes, a time sufficient to ensure thorough brushing.

### Example 6 (outside the scope of the invention)

This example illustrates how to prepare a toothpaste A wherein the flavor intensity of a second flavor signal is controlled. In this example, flavored chips from a three-layer film are prepared in accordance with the procedure of Example 1, except that the amount of flavor in the core layer is varied up to about 40%. The higher the flavor content of the core layer the stronger the flavor intensity of the second flavor signal of toothpaste A.

### Example 7 (outside the scope of the invention)

This example illustrates how to provide a second flavor signal that is different from the flavor signal of the base toothpaste. Flavored chips from a 3-layer film are prepared in accordance with the procedure of Example 1. The flavored chips are then added to a toothpaste having the composition of toothpaste A of Example 3 except that the flavor of toothpaste A is different from the flavor of the 3-layer flavored chips. Upon brushing with the toothpaste prepared according to this example, the consumer experiences a second flavor signal which is different and more intense from a first flavor signal of the base toothpaste. The second flavor signal is caused by the release of flavor from the 3-layer film chips following a pattern as shown in Figure 1.

### Example 8 (outside the scope of the invention)

This example illustrates how to prepare a toothpaste that can provide a cooling sensation signal in addition to a burst of flavor. Chips from a 3-layer film are prepared as in Example 1 except that the flavor of the center layer is supplemented with a cooling sensation agent such as WS-5. The WS-5-containing flavored chips are then added to a toothpaste having the composition of toothpaste A of Example 3. Upon brushing the consumer feels a cooling sensation in addition to the flavor of toothpaste A and the flavor present in the 3-layer chips contained therein.

### Example 9 (outside the scope of the invention)

This example shows how to prepare a toothpaste that can provide a tingling sensation signal in addition to a burst of flavor. Chips from a 3-layer film are prepared as in Example 1 except that the flavor of the center layer is supplemented with a tingling sensation agent such as spilanthol. The spilanthol containing flavored chips are then added to a toothpaste having the composition of toothpaste A of Example 3. Upon brushing a consumer feels a tingling sensation in addition to the flavor of toothpaste A and the flavor present in the 3-layer chips contained therein.

### Example 10 (outside the scope of the invention)

This example illustrates how to prepare a toothpaste having a sweet sensation signal in addition to a burst of flavor. Chips from a 3-layer film are prepared as in Example 1 except that the flavor of the center layer is supplemented with a sweet sensation agent such as sucralose. The flavored chips also containing a sweet sensation agent are then added to a toothpaste having the composition toothpaste A of Example 3. Upon brushing a customer feels a sweet sensation in addition to the flavor of toothpaste A and the flavor present in the 3-layer chips contained therein.

### Example 11 (outside the scope of the invention)

This example shows how to prepare a toothpaste having a warming sensation signal in addition to a burst of flavor. Chips from a three-layer film are prepared as in Example 1 except that the flavor of the center layer is supplemented with a warming sensation agent such as water-activated zeolites. The flavored chips also containing a warming sensation agent are then added to a toothpaste having the composition of toothpaste A of Example 3. Upon brushing, after the center layer is exposed to water from saliva, the user feels a warming sensation in addition to the flavor of toothpaste A and the flavor present in the 3-layer chips contained therein.

## Claims

1. An oral care composition having enhanced flavor release comprising
an orally acceptable carrier containing a first flavor; and
a multilayer film, the multilayer film including a center layer disposed between two outer surface layers, wherein at least one of said outer surface layers further comprises a release modulating agent which is polyvinyl acetate or hydroxyethyl cellulose,
wherein said center layer contains a second flavor,
wherein each layer comprises a film forming polymer selected from hydroxypropylmethyl cellulose, methyl cellulose, hydroxypropyl cellulose and mixtures of copolymers thereof, and
wherein the film forming polymer comprises from 25% to 75% by weight of the multilayer film.

2. The composition of Claim 1, wherein the release modulating agent comprises from 5% to 30% by weight of the multilayer film.

3. The composition of Claim 1, wherein the orally acceptable carrier is a dentifrice or a mouthwash.

4. The composition of Claim 1, wherein the thickness of each outer surface layer is from 2.54 µm to 101.6 µm (0.1 mils to 4 mils), optionally from 7.62 µm to 19.05 µm (0.3 mils to 0.75 mils).

5. The composition of Claim 1, wherein said second flavor comprises from 1% to 60%, by weight, of said center layer; optionally wherein said second flavor comprises from 5% to 40%, by weight, of said center layer; further optionally wherein said second flavor comprises from 10% to 25%., by weight, of said center layer.

6. The composition of Claim 1, wherein the center layer further comprises one or more agents selected from the group consisting of a cooling sensation agent; a tingling sensation agent; a sweet signal agent; a warming sensation agent; and mixture of two or more thereof.

7. The composition of Claim 6, wherein the cooling sensation agent is selected from the group consisting of menthol, N-ethyl-p-menthan-3-carboxamide, ethyl-3-(p-menthane-3-carboxamido)acetate, 2-isopropyl-N, 2, 3-trimethylbutyramide and L-menthyl lactate.

8. The composition of Claim 6, wherein the tingling sensation agent is selected from the group consisting of spilanthol, capsaicin, and capsicum oleoresin.

9. The composition of Claim 6, wherein the sweet signal agent is selected from the group consisting of sodium saccharin, sucralose, and

10. The composition of Claim 6, wherein the warming sensation agent is selected from the group consisting of cinnamic aldehye, zeolites, and capsaicin.

11. The composition of claim 1, wherein said first flavor and said second flavor are the same flavor.

12. The composition of claim 1, wherein said first flavor and said second flavor are different flavors.

13. The composition of claim 1, wherein said multilayer film further comprises additional layers of film.

## Patentansprüche

1. Mundpflegezusammensetzung mit erhöhter Aromafreisetzung, umfassend einen oral verträglichen Träger, der ein erstes Aroma enthält;
und
einen mehrschichtigen Film, wobei der mehrschichtige Film eine mittlere Schicht einschließt, die zwischen zwei äußeren Oberflächenschichten angeordnet ist, wobei mindestens eine der äußeren Oberflächenschichten weiterhin ein Freisetzung modulierendes Mittel umfasst, das Polyvinylacetat oder Hydoxyethylcellulose ist,
wobei die mittlere Schicht ein zweites Aroma enthält,
wobei jede Schicht ein filmbildendes Polymer umfasst, das aus Hydroxypropylmethylcellulose, Methylcellulose, Hydroxypropylcellulose und Gemischen von Copolymeren davon ausgewählt ist, und
wobei das filmbildende Polymer von 25% bis 75% bezogen auf das Gewicht des mehrschichtigen Films umfasst.

2. Zusammensetzung nach Anspruch 1, wobei das Freisetzung modulierende Mittel von 5% bis 30% bezogen auf das Gewicht des mehrschichtigen Films umfasst.

3. Zusammensetzung nach Anspruch 1, wobei der oral verträgliche Träger ein Zahnreinigungsmittel oder ein Mundwasser ist.

4. Zusammensetzung nach Anspruch 1, wobei die Dicke jeder äußeren Oberflächenschicht von 2,54 µm bis 101,6 µm (0,1 mils bis 4 mils), gegebenenfalls von 7,62 µm bis 19,05 µm (0,3 mils bis 0,75 mils) beträgt.

5. Zusammensetzung nach Anspruch 1, wobei das zweite Aroma von 1% bis 60% bezogen auf das Gewicht der mittleren Schicht umfasst; gegebenenfalls wobei das zweite Aroma von 5% bis 40% bezogen auf das Gewicht der mittleren Schicht umfasst; weiter gegebenenfalls wobei das zweite Aroma von 10% bis 35% bezogen auf das Gewicht der mittleren Schicht umfasst.

6. Zusammensetzung nach Anspruch 1, wobei die mittlere Schicht weiterhin ein oder mehrere Mittel umfasst, die aus der Gruppe bestehend aus einem als kühlend wahrgenommenen Mittel; einem als prickelnd wahrgenommenen Mittel; einem Süße signalisierenden Mittel; einem als wärmend wahrgenommenen Mittel; und einem Gemisch von zwei oder mehr davon ausgewählt sind.

7. Zusammensetzung nach Anspruch 6, wobei das als kühlend wahrgenommene Mittel aus der Gruppe bestehend aus Menthol, N-Ethyl-p-menthan-3-carboxamid, Ethyl-3-(p-menthan-3-carboxamido)acetat, 2-Isopropyl-N,2,3-trimethylbutyramid und L-Menthyllactat ausgewählt ist.

8. Zusammensetzung nach Anspruch 6, wobei das als prickelnd wahrgenommene Mittel aus der Gruppe bestehend aus Spilanthol, Capsaicin und Capsicum-Oleoresin ausgewählt ist.

9. Zusammensetzung nach Anspruch 6, wobei das Süße signalisierende Mittel aus der Gruppe bestehend aus Natriumsaccharin, Sucralose und ausgewählt ist.

10. Zusammensetzung nach Anspruch 6, wobei das als wärmend wahrgenommene Mittel aus der Gruppe bestehend aus Zimtaldehyd, Zeoliten und Capsaicin ausgewählt ist.

11. Zusammensetzung nach Anspruch 1, wobei das erste Aroma und das zweite Aroma das gleiche Aroma sind.

12. Zusammensetzung nach Anspruch 1, wobei das erste Aroma und das zweite Aroma verschiedene Aromen sind.

13. Zusammensetzung nach Anspruch 1, wobei der mehrschichtige Film weiterhin zusätzliche Schichten an Film umfasst.

## Revendications

1. Composition de soin buccal présentant une meilleure libération d'arôme comprenant
un support acceptable par voie orale contenant un premier arôme ; et
un film multicouche, le film multicouche comprenant une couche centrale disposée entre deux couches de surface externes, dans lequel au moins l'une desdites couches de surface externes comprend en outre un agent de modulation de libération qui est du poly(acétate de vinyle) ou de l'hydroxyéthylcellulose,
dans laquelle ladite couche centrale contient un deuxième arôme,
dans laquelle chaque couche comprend un polymère filmogène choisi parmi l'hydroxypropylméthylcellulose, la méthylcellulose, l'hydroxypropylcellulose et des mélanges de leurs copolymères, et
dans laquelle le polymère filmogène constitue de 25 % à 75 % en poids du film multicouche.

2. Composition selon la revendication 1, dans laquelle l'agent de modulation de libération constitue de 5 % à 30 % en poids du film multicouche.

3. Composition selon la revendication 1, dans laquelle le support acceptable par voie orale est un dentifrice ou un rince-bouche.

4. Composition selon la revendication 1, dans laquelle l'épaisseur de chaque couche de surface externe est de 2,54 µm à 101,6 µm (0,1 mils à 4 mils), éventuellement de 7,62 µm à 19,05 µm (0,3 mils à 0,75 mils).

5. Composition selon la revendication 1, dans laquelle ledit deuxième arôme constitue de 1 % à 60 %, en poids, de ladite couche centrale ; éventuellement dans laquelle ledit deuxième arôme constitue de 5 % à 40 %, en poids, de ladite couche centrale ; en outre éventuellement, dans laquelle ledit deuxième arôme constitue de 10 % à 25 %, en poids, de ladite couche centrale.

6. Composition selon la revendication 1, dans laquelle la couche centrale comprend en outre un ou plusieurs agents choisis dans le groupe constitué par un agent de sensation de rafraichissement ; un agent de sensation de picotements ; un agent de signal sucré ; un agent de sensation de réchauffement ; et un mélange de deux ou plus de ceux-ci.

7. Composition selon la revendication 6, dans laquelle l'agent de sensation de rafraichissement est choisi dans le groupe constitué par le menthol, le N-éthyl-p-menthane-3-carboxamide, l'éthyl-3-(p-menthane-3-carboxamido)acétate, le 2-isopropyl-N,2,3-triméthylbutyramide et le lactate de L-menthyle.

8. Composition selon la revendication 6, dans laquelle l'agent de sensation de picotement est choisi dans le groupe constitué par le spilanthol, la capsaïcine et l'oléorésine de capsicum.

9. Composition selon la revendication 6, dans laquelle l'agent de signal sucré est choisi dans le groupe constitué par la saccharine sodique, le sucralose, et.

10. Composition selon la revendication 6, dans laquelle l'agent de sensation de réchauffement est choisi dans le groupe constitué par l'aldéhyde cinnanique, les zéolites et la capsaïcine.

11. Composition selon la revendication 1, dans laquelle ledit premier arôme et ledit deuxième arôme sont le même arôme.

12. Composition selon la revendication 1, dans laquelle ledit premier arôme et ledit deuxième arôme sont des arômes différents.

13. Composition selon la revendication 1, dans laquelle ledit film multicouche comprend en outre des couches de film supplémentaires.
